# EUROPEAN PATENT SPECIFICATION

(11) **EP 2 762 069 B1**
(45) Date of publication and mention of the grant of the patent: **12.04.2017**
(21) Application number: 14152680.6
(22) Date of filing: 27.01.2014
(51) Int. Cl.: A61B 5/0478, A61B 5/0482, A61B 5/00

(54) **Brain biofeedback device with radially adjustable electrodes**
Gehirn-Biofeedbackvorrichtung mit radial einstellbaren Elektroden
Dispositif de rétroaction biologique du cerveau avec électrodes réglables radialement

(30) Priority: 31.01.2013 US 201313754919; 31.01.2013 CN 201310037752
(43) Date of publication of application: 06.08.2014
(73) Proprietor: The Hong Kong Polytechnic University, Innovation and Technology Development Office The Hong Kong Polytechnic University Hung Hom Kowloon Hong Kong (CN)
(72) Inventor: Tong, Kai Yu, Kowloon Hong Kong (CN); Yu, Bun, Kowloon Hong Kong (CN); Pang, Peter Man Kit, Kowloon Hong Kong (CN); Ho, Newmen Sze Kit, Kowloon Hong Kong (CN); Hu, Xiaoling, Kowloon Hong Kong (CN); Tam, Robert Wai Man, Kowloon Hong Kong (CN); Ng, Shu To, Kowloon Hong Kong (CN)
(74) Representative: Modiano, Micaela Nadia

(56) References cited:
- WO-A1-2011/123059
- DE-A1-102010 056 099
- US-A1- 2007 225 585
- US-A1- 2011 098 593
- US-A1- 2012 143 020

## Description

### Field of the Invention

The invention relates to an EEG brain biofeedback device for rehabilitation, training or entertainment and, more particularly, to a brain biofeedback device having self-orienting, radially-adjustable EEG electrodes.

### Background of the Invention

Stroke is a cerebrovascular accident with high disability and mortality rates. One of the main factors affecting the independence of stroke survivors is hand function, which is closely related to daily activities, such as feeding and self-cleaning. Neuro-rehabilitation following a stroke or other cerebrovascular event is a major future challenge as populations age and have increasing longevity.

Electroencephalography (EEG) is a technique for measuring bioelectrical signals generated by the cerebral cortex of a brain. The signals are directly related to voluntary motor contributions from the central nervous system (e.g., EEG motor imagery, the thinking and planning of a physical task). EEG signals are more directly related to the voluntary contribution with stronger signals from a patient in the early post-stroke stage. Currently the types of EEG systems that can measure these signals are non-portable, that is, they are sufficiently large as to restrict their use to a research laboratory environment. The measurement system requires a lengthy period to prepare and correctly position all the EEG electrodes. Further, without visual indicators, correct electrode placement is difficult to verify and typically must be performed by skilled technicians.

Various devices have been used in an attempt to correctly position and hold EEG electrodes adjacent to a patient's scalp. For example, caps are used to position the EEG electrodes. Such electrode caps facilitate positioning of EEG electrodes by technicians within a short period of time (e.g., about 5 minutes). Following electrode positioning, conductive gel is injected to reduce the scalp-electrode impedance and thereby record strong EEG signals.

However, conventional attempts to position EEG electrodes using various headgear are insufficient because they do not appropriately account for variations among head sizes and shapes in the patient population. Typically, conventional approaches use several specific sizes in order to approximate various head sizes along with elastic materials to roughly elongate a cap to more closely fit different head shapes. However, conventional electrode caps are based on approximating the upper head as having a hemispherical shape. Since the human head does not have a hemispherical shape, an equal elongated head size approximation method causes error in the electrode positioning.

Thus there is a need in the art for an improved EEG electrode positioning device, particularly a positioning device that is lightweight with sufficient resilient properties to ensure proper electrode positioning on a variety of head sizes and shapes. There is a further need in the art for visual indication that the electrodes are correctly positioned and that the electrode-scalp impedance is within an acceptable range for EEG signal measurement. Such a device could facilitate a portable brain-training system with minimal set-up time that could be used in clinical and residential settings.

Examples of head-mountable devices including an electrode array for measuring bioelectrical signals generated by a cerebral cortex of a brain are disclosed in German Patent Application DE102010056099 A1 (UNIV ILMENAU TECH) 21.06.2012 and PCT Application WO2011/123059 A1 (Agency for Science, Technology and Research) 06.10.2011.

German Patent Application DE102010056099 A1 describes an apparatus and associated method which put sensors on the surface of a biological object. One of the main problems in using this apparatus is to wear the apparatus while keeping the sensors in correct position. The apparatus is a one-piece device which cannot be put on the surface of the biological object in forms of different sections. The sensors will then be displaced from the correct position and the orientation as they move along the surface of the biological object during the time when the apparatus is being put on.

PCT Application WO2011/123059 A1 relates to a non-invasive EEG-based brain-computer interface. The brain-computer interface is proposed to acquire EEG signal from a person by detecting motor imagery signals to control robotic device for rehabilitation. One of the challenges in applying this brain-computer interface in clinical use is the long setup time to have all the EEG electrodes in the correct position on the scalp. Another challenge is the long skin preparation time for each EEG electrode owing to the absence of visual indicators from the sensor device to show the impedance between the sensor and the skin.

### Summary of the Invention

The present invention is disclosed in independent claim 1 and presents a novel head-mountable EEG electrode-containing device based on radially adjustable electrodes to fit the wearer's unique head size and shape rather than merely laterally elongating the space between electrodes as in conventional electrode caps.

A head-mountable device with an electrode array positioned therein includes multiple head-mountable device sections that are interconnected by mechanical fasteners to facilitate sizing and positioning of the head-mountable device. An array of resilient sleeves is positioned within each head-mountable device section. Each resilient sleeve houses an individual electrode and is deformable. The deformation of the sleeve is such that a central axis passing through the individual electrode housed within the resilient sleeve is maintained in a position approximately normal to a plane tangential to a scalp portion positioned beneath that electrode.

### Brief Description of the Drawings

FIG. 1 is a perspective view of a brain training device consisted of EEG headset module in accordance with a preferred embodiment of the present invention as worn.
FIG. 2 schematically depicts expansion of a head dimension in a radial direction based on measurements of human populations.
FIGS. 3A and 3B depict top and side views of positioning points for the head-mountable device electrode array of the present invention.
FIGS. 4A and 4B are, respectively, front and side views of the EEG headset module of the device of FIG. 1.
FIG. 5 is a top view of the EEG headset module of the device of FIG. 1 indicating the EEG electrode positions which covered the central, frontal and parietal regions of the brain activity based on the EEG electrode location from the International 10/20 system for a 64 channel EEG.
FIG. 6 is a cross-sectional view illustrating details of a self-orienting EEG electrode attached inside the headset module of the device of FIG. 1.
FIGS. 7A and 7B are cross-sectional views of a self-orienting EEG electrode of FIG. 6 in contact with the skin surface with a certain distance and angle that the electrode can be placed on the various size and shape of the head. FIG. 7B shows the self-orienting properties of the electrode with a central axis passing through the electrode maintained in a position approximately normal to a plane tangential to the portion of the scalp positioned beneath the electrode.
FIG. 8 is a front view of the EEG headset module of the device of FIG. 1 when both left and right segments of the EEG headset module are bent upward for placing the device on the head or removing the device from the head.
FIG. 9 is a front view of the EEG headset module of the device of FIG. 1 when both left and right segments of the EEG headset module are attached on the head surface.
FIGS. 10A and 10B are rear perspective views of the EEG headset module of the device of FIG. 1 showing both left and right reference electrodes adjusted along the axis when the device is worn.
FIG. 11 is the top view of the self-orienting EEG electrode of FIG. 6 showing a diffused illumination pattern of the light ring for the visual bio-feedback signal.
FIG. 12 is a top view of the EEG headset module of the device of FIG. 1 indicating the diffused illumination color pattern on the visual signal indicator of FIG. 11 which placed outside of the device.

### Detailed Description of the Invention

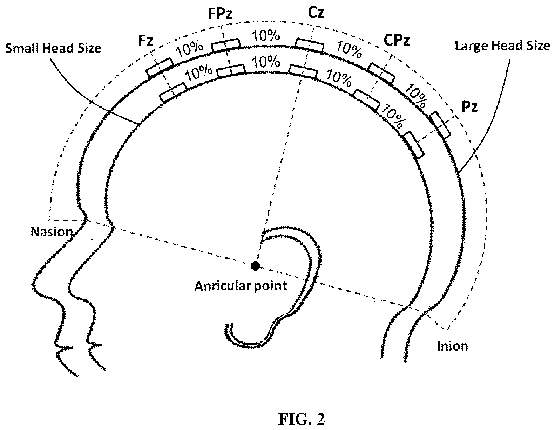

Turning to the drawings in detail, FIG. 2 depicts an extension of a head size in a radial direction obtained by comparing standard head shapes and sizes (see references 1-6) according to the present invention. Using these measurements and extensions, the present invention creates a new design for a head-mountable electrode array device. The device relies on radially adjustable electrodes that fit an individual head size in contrast to conventional elastic caps that laterally elongate the space between the electrodes.

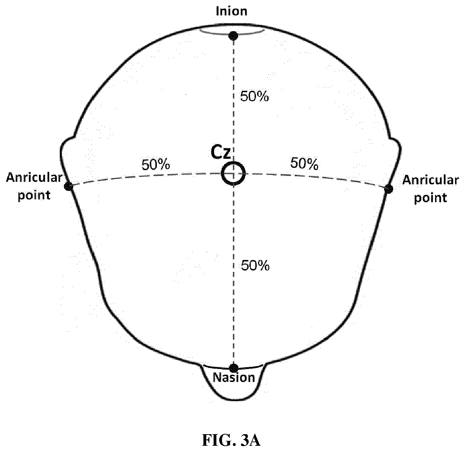

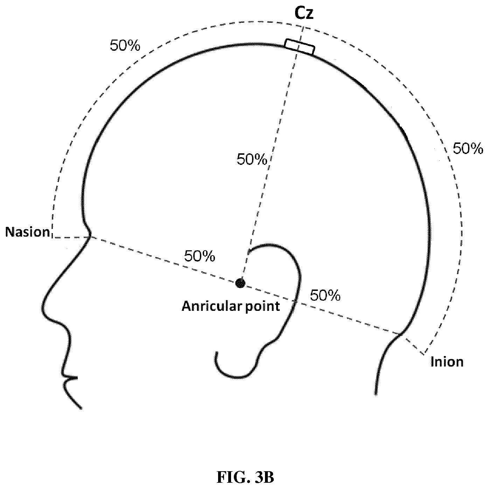

Turing to FIG. 3, the head-mountable device is attached on the wearer's head using the precise orientation and position with reference to the nasion and inion, and also the ear auricular points to find the vertex point (Cz), which is the central point between nasion and inion and also the central point between left and right auricular points. The device is also aligned with the central line, which runs along the nasion and inion.

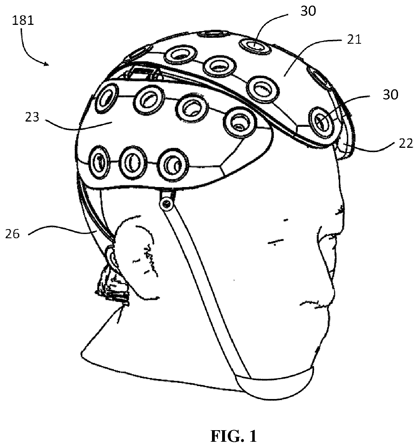

Based on the measurements and positions of FIGS. 2 and 3, FIG. 1 depicts a head-mountable device **181** that can measure bioelectrical signals generated by a cerebral cortex of a brain configured such that individual electrodes are radially adjustable for individual variations in head size and shape. The device of FIG. 1 is optionally part of a system used for brain training and rehabilitation.

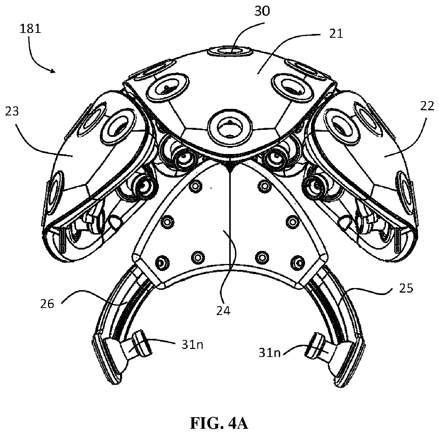

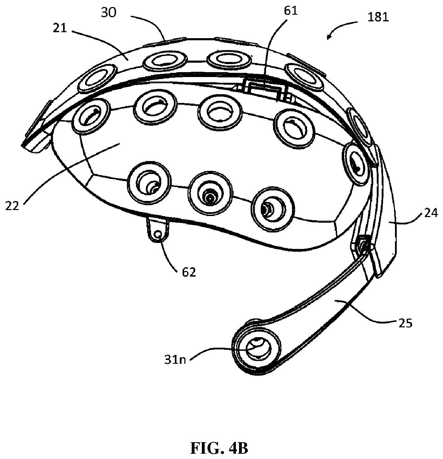

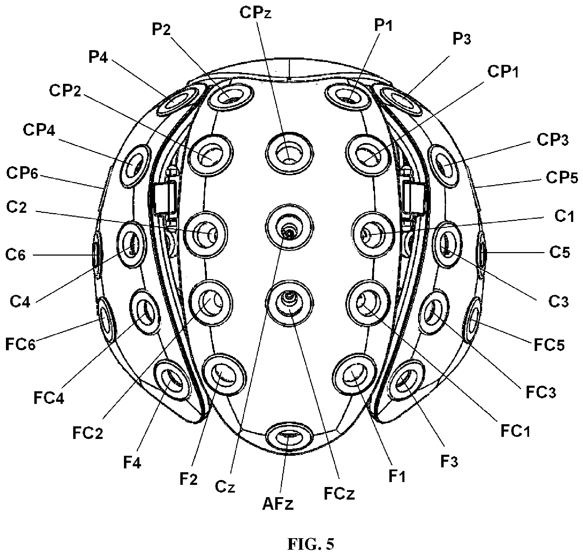

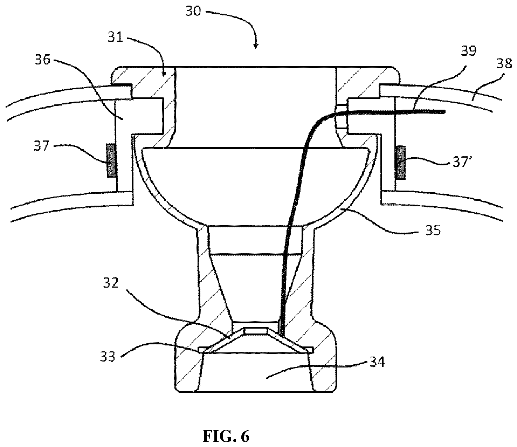

Referring to FIG. 4 and 5, the EEG head-mountable device **181** includes EEG electrode positions **30** covering the central, frontal and parietal regions of the skull of the head according to the international 10/20 system. The numeral **30** is used to indicate the overall electrode structure while the bioelectrical signal sensing portion (the actual electrical portion of the electrode) is indicated by reference numeral **32** (see FIG. 6). The head-mountable device **181** comprises central device segment **21,** left device segment **22,** right device segment **23** and auxiliary device segment **24.** The auxiliary segment **24** includes left and right back ear reference electrodes **31n**. Each of the EEG electrodes **30** is affixed to a predetermined electrode position in the EEG head-mountable device. An electrode is placed with spacing of 20% of the nasion-inion distance and 20% of the ear auricular distance for the vertical and horizontal line respectively according to the standard 10/20 system. For a 64-channel EEG, the vertical distance between electrodes is reduced by half and becomes 10% of the nasion-inion distance and the horizontal distance becomes 10% of the ear auricular distance.

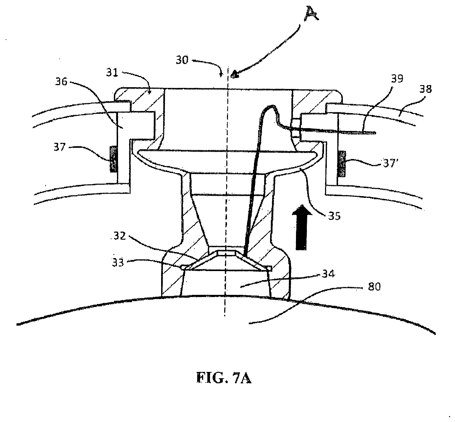

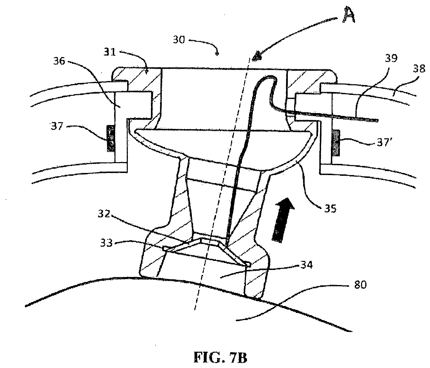

FIG. 6 is a cross-sectional view of an electrode **30.** The bioelectrical sensing portion **32** is positioned within a resilient electrode sleeve **31.** The resilient electrode sleeve **31** is optionally an integrally-formed that includes semicircular damper portion **35.** This configuration allows the resilient sleeve **31** to be compressed within a certain distance to change its orientation to adapt to various head sizes and shapes as shown in FIGS. **7A** and **7B****.** As seen in FIGS. 7A and 7B, the electrode **32** is mounted within resilient sleeve **31** such that when the resilient sleeve with damper portion **35** is deformed, a central axis a passing through electrode **32** is maintained in a position approximately normal to a plane tangential to a scalp portion positioned beneath the electrode for self-orienting.

The EEG electrode **32** is positioned within the sleeve having distal ends/legs embedded in the annular groove **33** of the resilient sleeve **31.** Embedding the EEG sensing electrode **32** inside the self-orienting electrode sleeve **31** and fixing the orientation of cable **39** minimizes the possibility of EEG electrode dislocation.

At the portion of the resilient sleeve **31** directly adjacent to a patient's scalp, a cavity **34** is formed. This cavity is typically filled with a conductive gel to provide contact between the scalp and the electrode. Alternatively, a deformable conductive material can fill cavity **34** for provide the needed skin-electrode impedance. The annular edge of the resilient sleeve **31** can also be placed on the head comfortably and minimize the leakage from the electrode during head movement. The conductive gel can reduce the skin-electrode impedance between the EEG electrode **32** and scalp **80** of the head. This design can reduce the skin-electrode impedance to enhance the EEG signal quality.

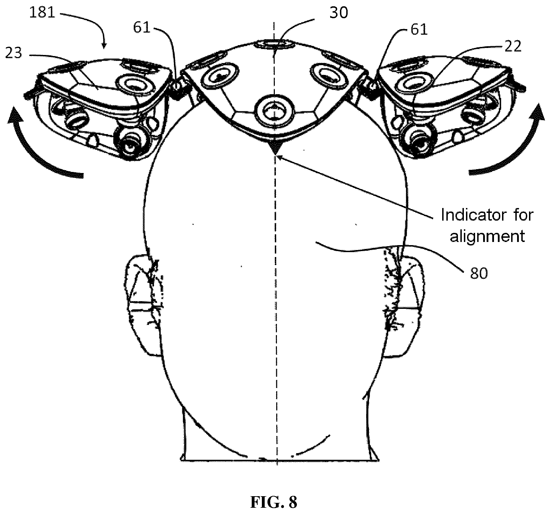

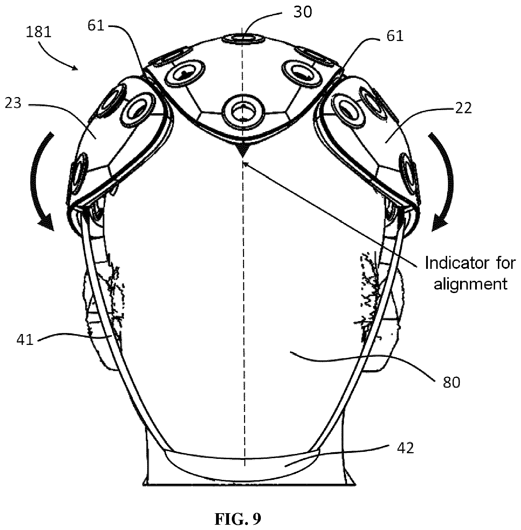

Referring to FIGS. 8 and 9, the left segment **22** and right segment **23** of the EEG head-mountable device **181** can be bent upward and downward by mechanical fasteners (optionally formed by a hinge joint **61**) during application and removal of device **181.** Advantageously, the relative movement of the sections to one another avoids deforming the electrode at the left segment **22** and right segment **23** of the EEG head-mountable device during application of the device. However, the electrode sleeve **31** will deform radially and self-orient at the desired electrode position on the patient's head (that is, with the central axis of the electrode substantially perpendicular to a line tangent to the scalp beneath the electrode).

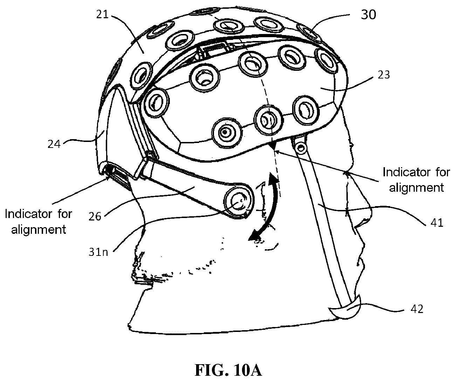

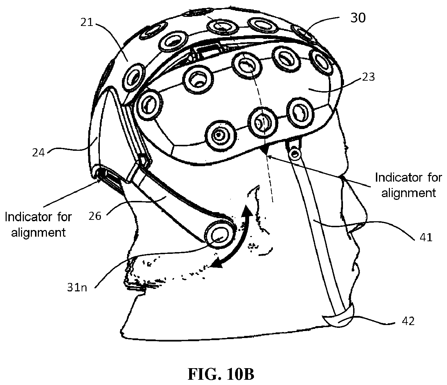

FIG. 10 depicts left reference electrode **25** and right reference electrode **26** that are located at the auxiliary segment **24** of the head-mountable device **181.** The left reference electrode **25** and right reference electrode **26** can be adjusted along the axis at a selected angle to contact with the ear reference location.

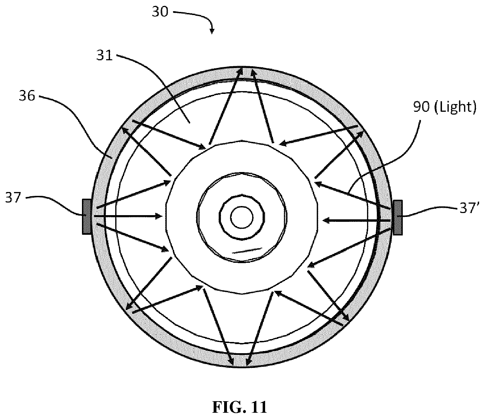

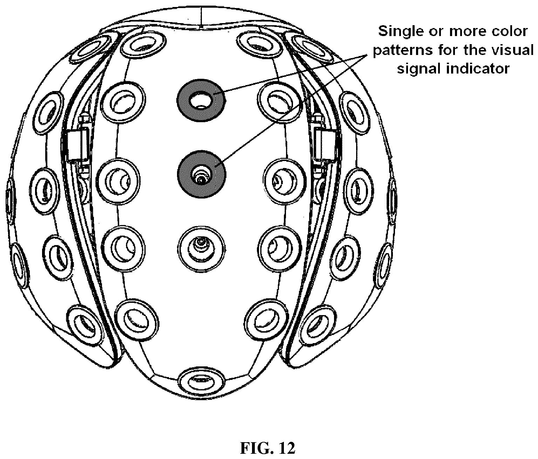

Referring to FIG. 11 and FIG. 12, the visual bio-feedback signal indicators **30** are incorporated in the electrode design to diffuse a color pattern on the light ring which is placed outside of the device. The visual signal indicators can show the skin-electrode impedance value, the EEG value, or control parameters. The visual signal indicator can be one or more LEDs **37** with single or more colors. The color patterns and intensity represent impedance, EEG value or control parameters. The color is not limited to cover only the above pattern and parameters.

In order to provide better signal-to-noise ratio for the EEG signals, a pre-amplifier can be attached physically close to each electrode. The amplifier can be arranged in a single unit or in the form of an array.

As set forth above, the present invention provides an improved biofeedback device that is portable, easy-to-use, and minimizes the preparation time for brain training both in a hospital and home setting. While the foregoing invention has been described with respect to various embodiments and examples, it is understood that other embodiments are within the scope of the present invention as expressed in the following claims and their equivalents. Moreover, the above specific examples are to be construed as merely illustrative, and not limitative of the remainder of the disclosure. The following references are useful for easy understanding of the background of the invention.

### References

1. R. Ball, C. Shu, P. C. Xi, M. Rioux, Y. Luximon, and J. Molenbroek, "A comparison between Chinese and Caucasian head shapes," Applied Ergonomics, vol. 41, pp. 832-839, 2010
2. Z. Zhuang, S. Benson, D. "Viscusi. Digital 3-D headforms with facial features representative of the current U.S. work force," Ergonomics; 53: 661-71, 2010
3. China National Institute of Standardization. (1998) CNIS GB/T2428:1998. Head-face dimensions of adults by Xiao H, Hua DH, Yang TX, Zhang ZB, Bi GX, Liu JM. Beijing, China: General Administration of Quality Supervision, Inspection and Quarantine of the People's Republic of China.
4. China National Institute of Standardization. (1998) CNIS GB/T2428:1998. Head-face dimensions of adults by Xiao H, Hua DH, Yang TX, Zhang ZB, Bi GX, Liu JM. Beijing, China: General Administration of Quality Supervision, Inspection and Quarantine of the People's Republic of China.
5. China National Institute of Standardization. (1981) CNIS GB2428-81. Head styles of adults by Beijing Institute of Labor Protection. Beijing, China: General Administration of Quality Supervision, Inspection and Quarantine of the People's Republic of China.
6. Y. Yu, S. Benson, W. Cheng, J. Hsiao, Y. Liu, Z. Zhuang and W. Chen. "Digital 3-D Headforms Representative of Chinese Workers" Ann. Occup. Hyg., pp. 1-10, 2011

## Claims

1. A head-mountable device (181) including an electrode array for measuring bioelectrical signals generated by a cerebral cortex of a brain configured such that individual electrodes (30) are radially adjustable for individual variations in head size and shape comprising:
a plurality of head-mountable device sections (21, 22, 23, 24) that can be bent upward and downward by mechanical fasteners during application and removal of device (181) such that the device sections are moveable relative to one another;
an array of resilient sleeves (31) within each head-mountable device section (21, 22,23,24),
wherein each resilient sleeve (31) houses an individual electrode (30), the resilient sleeve (31) being deformable for self-orienting such that a central axis (A) passing through a bioelectrical signal sensing portion (32) of the individual electrode (30) housed within the resilient sleeve (31) is maintained, in use, in a position approximately normal to a plane tangential to a scalp portion (80) positioned beneath the electrode (32); and
wherein each resilient sleeve (31) further comprises a deformable damping portion (35) that is semicircular in a cross section and configured to be deformable for maintaining the orientation of the individual electrode (30) relative to the scalp portion (80) beneath the individual electrode (30) so as to achieve said self-orienting; wherein the damping portion (35) allows each resilient sleeve (31) to be compressed within a certain distance to change its orientation to adapt to various head sizes and shapes.

2. The head-mountable device (181) according to claim 1 wherein the mechanical fasteners comprise one or more hinge joints (61).

3. The head-mountable device (181) according to claim 1 further comprising a visual indicator to display information regarding an impedance value, an EEG value, or a control parameter.

4. The head-mountable device (181) according to claim 3 wherein the visual indicator comprises one or more light-emitting diodes (37).

5. The head-mountable device (181) according to claim 1 further comprising a pre-amplifier adjacent to the electrode (32).

6. The head-mountable device (181) according to claim 5 wherein the pre-amplifier is a single unit or an array.

7. The head-mountable device (181) according to claim 1 further comprising an auxiliary head-mountable segment (24) including reference electrodes (25, 26) alignable with an ear reference location.

8. The head-mountable device (181) according to claim 1 further comprising an annular groove (33) positioned within the resilient sleeve (31) for holding electrode legs (32).

9. A system for neuro-rehabilitation involving brain biofeedback comprising the head-mountable (181) of claim 1.

## Patentansprüche

1. Eine am Kopf montierbare Vorrichtung (181), die eine Elektrodenanordnung zur Messung bioelektrischer Signale einschließt, welche von einer Großhirnrinde eines Gehirns erzeugt werden, derart konfiguriert, dass einzelne Elektroden (30) für individuelle Variationen der Kopfgröße und -form radial verstellbar sind; Folgendes umfassend:
eine Vielzahl am Kopf montierbarer Vorrichtungsabschnitte (21, 22, 23, 24), die während der Anbringung und Abnahme der Vorrichtung (181) durch mechanische Befestigungselemente nach oben und nach unten gebogen werden können, so dass die Vorrichtungsabschnitte im Verhältnis zueinander beweglich sind;
eine Anordnung elastischer Hülsen (31) innerhalb jedes am Kopf montierbaren Vorrichtungsabschnitts (21, 22, 23, 24),
wobei jede elastische Hülse (31) eine einzelne Elektrode (30) aufnimmt, die elastische Hülse (31) zur Selbstausrichtung verformbar ist, so dass eine Mittelachse (A), die durch einen bioelektrischen Signalerfassungsabschnitt (32) der einzelnen Elektrode (30) verläuft, die in der elastischen Hülse (31) untergebracht ist, im Gebrauch in einer Position gehalten wird, die ungefähr senkrecht zu einer Ebene tangential zu einem Kopfhautabschnitt (80) ist, der sich unterhalb der Elektrode (32) befindet; und
wobei jede elastische Hülse (31) weiter einen verformbaren Dämpfungsabschnitt (35) umfasst, der einen halbkreisförmigen Querschnitt hat und ausgebildet ist, um verformbar zu sein, um die Ausrichtung der einzelnen Elektrode (30) relativ zum Kopfhautabschnitt (80) unterhalb der einzelnen Elektrode (30) beizubehalten, um die Selbstausrichtung zu erzielen; wobei der Dämpfungsabschnitt (35) das Zusammendrücken jeder elastischen Hülse (31) innerhalb einer gewissen Distanz ermöglicht, um ihre Ausrichtung zum Zwecke der Anpassung an verschiedene Kopfgrößen und -formen zu verändern.

2. Die am Kopf montierbare Vorrichtung (181) gemäß Anspruch 1, wobei die mechanischen Befestigungselemente eine oder mehrere Scharnierverbindungen (61) umfassen.

3. Die am Kopf montierbare Vorrichtung (181) gemäß Anspruch 1, die weiter eine visuelle Anzeige umfasst, um Informationen bezüglich eines Impedanzwertes, eines EEG-Wertes oder eines Steuerparameters anzuzeigen.

4. Die am Kopf montierbare Vorrichtung (181) gemäß Anspruch 3, wobei die visuelle Anzeige eine oder mehrere Leuchtdioden (37) umfasst.

5. Die am Kopf montierbare Vorrichtung (181) gemäß Anspruch 1, die weiter einen Vorverstärker neben der Elektrode (32) umfasst.

6. Die am Kopf montierbare Vorrichtung (181) gemäß Anspruch 5, wobei der Vorverstärker eine einzelne Einheit oder ein Array ist.

7. Die am Kopf montierbare Vorrichtung (181) gemäß Anspruch 1, die weiter ein zusätzliches am Kopf montierbares Segment (24) einschließlich Bezugselektroden (25, 26) umfasst, die mit einem Ohr-Bezugspunkt ausgerichtet werden können.

8. Die am Kopf montierbare Vorrichtung (181) gemäß Anspruch 1, die weiter eine Ringnut (33) umfasst, welche zum Halten von Elektrodenschenkeln in der elastischen Hülse (31) positioniert

9. Ein System zur Neuro-Rehabilitation, das Gehirn-Biofeedback beinhaltet, welches die am Kopf montierbare Vorrichtung (181) gemäß Anspruch 1 umfasst.

## Revendications

1. Dispositif (181) pouvant être monté sur la tête, comprenant un ensemble d'électrodes destinées à mesurer des signaux bioélectriques générés par le cortex cérébral du cerveau, conçu de telle sorte que des électrodes individuelles (30) sont réglables radialement pour des variations individuelles de taille et de forme de tête, comprenant :
une pluralité de sections (21, 22, 23, 24) de dispositif pouvant être monté sur la tête, qui peuvent être courbées vers le haut et vers le bas par des attaches mécaniques lors de l'application et du retrait du dispositif (181) de sorte que les sections de dispositif sont mobiles les unes par rapport aux autres ;
un ensemble de manchons élastiques (31) à l'intérieur de chaque section (21, 22, 23, 24) de dispositif pouvant être monté sur la tête,
dans lequel chaque manchon élastique (31) loge une électrode individuelle (30), le manchon élastique (31) pouvant être déformé pour permettre une orientation automatique de telle sorte qu'un axe central (A) traversant une partie de détection de signal bioélectrique (32) de l'électrode individuelle (30) logée à l'intérieur du manchon élastique (31) est maintenu, lors de l'utilisation, dans une position à peu près perpendiculaire à un plan tangent à une partie de cuir chevelu (80) placée sous l'électrode (32) ; et
dans lequel chaque manchon élastique (31) comporte en outre une partie d'amortissement déformable (35) qui présente une section transversale semi-circulaire et est conçue pour être pouvoir être déformée afin de maintenir l'orientation de l'électrode individuelle (30) par rapport à la partie de cuir chevelu (80) placée sous l'électrode individuelle (30) de manière à permettre ladite orientation automatique ; où la partie d'amortissement (35) permet à chaque manchon élastique (31) d'être comprimé sur une certaine distance pour changer son orientation afin de s'adapter à diverses tailles et formes de tête.

2. Dispositif (181) pouvant être monté sur la tête, selon la revendication 1, dans lequel les attaches mécaniques comprennent une ou plusieurs articulations (61).

3. Dispositif (181) pouvant être monté sur la tête, selon la revendication 1, comprenant en outre un indicateur visuel pour afficher des informations concernant une valeur d'impédance, une valeur EEG (électroencéphalogramme), ou un paramètre de commande.

4. Dispositif (181) pouvant être monté sur la tête, selon la revendication 3, dans lequel l'indicateur visuel comporte une ou plusieurs diodes électroluminescentes (37).

5. Dispositif (181) pouvant être monté sur la tête, selon la revendication 1, comprenant en outre un préamplificateur adjacent à l'électrode (32).

6. Dispositif (181) pouvant être monté sur la tête, selon la revendication 5, dans lequel le préamplificateur est une unité simple ou un ensemble.

7. Dispositif (181) pouvant être monté sur la tête, selon la revendication 1, comprenant en outre un segment auxiliaire (24) pouvant être monté sur la tête, comportant des électrodes de référence (25, 26) pouvant être alignées avec une position de référence d'oreille.

8. Dispositif (181) pouvant être monté sur la tête, selon la revendication 1, comprenant en outre une rainure annulaire (33) placée à l'intérieur du manchon élastique (31) afin de maintenir des pattes d'électrode (32).

9. Système de neuro-réhabilitation entraînant une rétroaction biologique du cerveau, comprenant le dispositif (181) pouvant être monté sur la tête, selon la revendication 1.
